(19) **Europäisches Patentamt**

**European Patent Office**

**Office européen des brevets**

(11) **EP 0 635 218 B1**

(12) **EUROPEAN PATENT SPECIFICATION**

(45) Date of publication and mention
of the grant of the patent:
**25.10.2000 Bulletin 2000/43**

(21) Application number: **94905856.4**

(22) Date of filing: **04.02.1994**

(51) Int. Cl.[7]: **A23L 1/03**, A61K 7/00,
A61K 7/16, A61K 47/48,
A61K 9/00, A61K 47/24,
A61K 47/42

(86) International application number:
**PCT/JP94/00176**

(87) International publication number:
**WO 94/17675 (18.08.1994 Gazette 1994/19)**

(54) **TASTE MODIFYING METHOD AND BITTER TASTE REDUCING METHOD**

VERFAHREN ZUR GESCHMACKSMODIFIZIERUNG UND VERFAHREN ZUR VERMINDERUNG EINES BITTEREN GESCHMACK

PROCEDE DE MODIFICATION DU GOUT ET PROCEDE D'ATTENUATION DE L'AMERTUME

(84) Designated Contracting States:
**CH DE FR GB LI**

(30) Priority: **05.02.1993 JP 1900093**
**09.02.1993 JP 2128593**

(43) Date of publication of application:
**25.01.1995 Bulletin 1995/04**

(73) Proprietors:
• **Kao Corporation**
**Chuo-Ku Tokyo 103 (JP)**
• **KURIHARA, Kenzo**
**Setagaya-ku, Tokyo 158 (JP)**

(72) Inventors:
• **KURIHARA, Kenzo**
**Setagaya-ku, Tokyo 158 (JP)**
• **KASHIWAGI, Mitsuyoshi**
**Matsudo-shi, Chiba 271 (JP)**
• **YASUMASU, Takeshi**
**Kashima-gun, Ibaraki 314-03 (JP)**
• **MITSUI, Yuuki**
**Kashima-gun, Ibaraki 314-03 (JP)**

• **INAOKA, Setsujiro**
**Kashima-gun, Ibaraki 314-03 (JP)**
• **KATSURAGI, Yoshihisa**
**Kashima-gun, Ibaraki 314-03 (JP)**

(74) Representative:
**Bannerman, David Gardner**
**Withers & Rogers,**
**Goldings House,**
**2 Hays Lane**
**London SE1 2HW (GB)**

(56) References cited:
**EP-A- 0 518 608**       **JP-A- 4 079 843**
**JP-A- 4 235 136**       **JP-A- 4 327 526**
**JP-A- 62 058 960**      **JP-A- 63 283 735**

• **PATENT ABSTRACTS OF JAPAN vol. 017, no. 693 (C-1144), 17 December 1993 & JP 05 236896 A (KAO CORP), 17 September 1993, & DATABASE WPI Week 9342 Derwent Publications Ltd., London, GB; AN 93-330534 & JP 05 236 896 A (KAO CORPORATION) , 17 September 1993**

## Description

The present invention relates to a flavour-modifying agent which removes or masks unpleasant tastes such as bitterness in a substance which is to be orally ingested, such as a foodstuff or a pharmaceutical, by imparting a pleasant flavour to the substance when the agent is added to it, and to a method of modifying the taste of a foodstuff or of a pharmaceutical product with the flavour-modifying agent.

Further, the present invention relates to a bitterness-decreasing or masking agent which can decrease an unpleasant bitter taste in a foodstuff, a pharmaceutical product or a cosmetic by adding the agent thereto, and a method for decreasing bitterness by means of the bitterness-decreasing agent.

Bitter-tasting substances are generally repellent substances and are often substances which a healthy person should not ingest. A human being has a biophylactic mechanism for sensing and rejecting bitter substances in his mouth, and a bitter-tasting substance can be sensed even if it is present only in an extremely small amount as compared with other substances such as sweet-, salty- and sour-tasting substances.

However, bitter-tasting substances are present in nearly all edible natural substances and human beings have learned that they are safe by experience. Further, human beings enjoy the bitterness of many foods for example, luxury items such as coffee, tobacco and tea or fermented foodstuffs such as beer and cheese. Thus, although bitterness is an important factor in the satisfaction obtained from food, the existence thereof can cause a foodstuff, a pharmaceutical product or a cosmetic to be rated low in quality.

The presence of an unpleasant bitter taste is a serious matter to the food industry. For example, the bitterness of amino acids or peptides present in the products of proteolysis and that of a bitter-tasting substance present in fruit juice lowers the perceived quality of foodstuffs. Methods of removing bitter-tasting substances from foods include the use of adsorbents or clathrate compounds and the addition of sweetening agents. However, these methods are unsatisfactory in that the bitterness cannot be completely eliminated and in that the taste of the food is altered, for example.

Bitterness is also a significant problem in the pharmaceutical industry. More precisely, nearly all of the pharmaceuticals used as medicines have a bitter taste and decreasing of the bitter taste has become a significant issue. In particular, the decreasing of the bitter taste is important in preparations for babies and small children. It is difficult for babies and children to take a solid or powdered preparation orally, so that a liquid preparation such as a syrup generally prepared. However, it is very difficult to decrease the bitterness of a liquid preparation. It has been a practice in the prior art to add a sweetening agent and a flavouring agent or an organic acid such as citric acid to decrease the bitterness of a liquid preparation. Further, the development of a pharmaceutical preparation having an easy-to-take dosage form has become expected for the aged, so that studies have been made particularly on the miniaturization of solid preparations, dosage forms which permit oral intake without using water and liquid preparations. Simultaneously studies have been made also for the purpose of decreasing bitterness.

Under these circumstances, methods such as microencapusulation, use of a coating agent soluble in the stomach, addition of a clathrate compound and chemical modification have been proposed as methods for decreasing the bitterness of pharmaceuticals, in addition to the above methods for decreasing the bitterness of liquid preparations. However, there is a problem that bitterness cannot be completely eliminated or that the range of pharmaceuticals to which any particular method can be applied is limited. Thus, no effective method of decreasing the bitterness of pharmaceuticals has been found as yet.

Cosmetics for the face and the mouth also tend to have a bitter taste. In particular, it is desirable that face lotions, mouthwashes and dentifrices be free from any bitter taste. However, some of the surfactants and flavours used therein have a bitter taste, so that the kinds and amounts of such compounds that can be used are often limited.

Although it has been a practice to attenuate the bitter taste of such cosmetics by the addition of a sweetening agent or a flavouring material as in the case of decreasing the bitterness of foods and pharmaceuticals, the effect attainable by such means is insufficient for cosmetics containing a strongly bitter component. With respect to cosmetics for the face and oral cavity, the components which are added in order to decrease the bitter taste are not always safely acceptable for oral ingestion. Thus, no cosmetic product having a sufficiently decreased bitterness has been proposed as yet.

Substances which modify the taste receptors have been described as seasoning agents or masking agents. For example, it is known that gymnetic acid from the leaves of certain asclepiadaceous plants and zizyphine from the leaves of the jujube, both of which are triterpene glycosides, have a depressing effect upon bitterness. Further, it has been found that miraculin obtained from the miracle fruit (a fruit of a sapotaceous plant) and curculin from a hypoxidaceous plant gives a sweet taste when the person holds it in his or her mouth and thereafter drinks a sour or tasteless liquid ( Kagaku to Seibutsu: Chemistry and Biology), Vol. 27, No. 6, p.p. 350-352, published on June 25, 1989, edited by the Agricultural Chemical Society of Japan). However, they are in short supply, difficult to separate and purify, chemically unstable and expensive, so have not found wide use industrially.

Accordingly, an object of the present invention is to provide, at a low cost, a taste-modifying agent or bitterness-decreasing agent which modifies the taste of foodstuffs, pharmaceutical products and cosmetics, and which is safe when ingested orally.

A bitterness inhibitor which has been studied by Katsuragi and Kurihara has been reported in Nature (Vol. 365, p.p. 213-214, published on September 16, 1993) and in the Asahi Newspaper (dated July 7, 1993).

The present inventors have directed their attention to the taste receptors located on the taste buds present on the tongue and the interaction between a substance having a taste and the taste receptors and have made extensive studies on the modification of flavours and reduction in bitterness. As a result, they have found that the protein-lipid complex according to the present invention adheres to the neighbourhood of the bitterness-receptor in the taste-receiving membrane present on the taste bud, thus inhibiting the perception of a bitter taste, and that simultaneously with this phenomenon or independently thereof, the complex adsorbs hydrophobic bitter-tasting substances, thus lowering their concentration in the mouth. The present invention has been accomplished on the basis of these findings.

Thus, the present invention provides an ingestible protein-lipid complex which is useful as a taste-modifying agent, and/or a bitterness-masking agent and/or a bitterness-decreasing agent.

The protein-lipid complex of the present invention can easily be mass-produced, can be provided at a low cost, is safe even when orally ingested, and can both decrease bitterness and increase sweetness and flavour.

Further, the present invention provides a method for decreasing bitterness which is characterised by holding the above protein-lipid complex in the oral cavity before and/or simultaneously with the oral ingestion of a bitter-tasting foodstuff, pharmaceutical or cosmetic to thereby mask the bitterness-receptors present in the oral cavity, a method for decreasing a bitterness of a foodstuff, pharmaceutical or cosmetic which comprises adding the above protein-lipid complex to a bitter-tasting food, pharmaceutical or cosmetic, and a method for modifying the taste of a foodstuff, pharmaceutical or cosmetic which comprises adding the above protein-lipid complex to a foodstuff, pharmaceutical or cosmetic.

Furthermore, the present invention provides the use of the above protein-lipid complex in decreasing the bitterness of a bitter-tasting foodstuff, pharmaceutical or cosmetic, and the use of the above protein-lipid complex in modifying the taste of a foodstuff, pharmaceutical or cosmetic.

The present invention provides a process for the preparation of a food composition, a medicinal composition or a cosmetic having a decreased bitterness which comprises adding the above protein-lipid complex to a bitter-tasting foodstuff, pharmaceutical or cosmetic, and a process for the preparation of a food composition, a pharmaceutical composition or a cosmetic having a modified taste which comprises adding the above protein-lipid complex to a food, pharmaceutical or cosmetic.

The present invention provides a food composition comprising a food and the above protein-lipid complex, a cosmetic comprising a cosmetic component and the above protein-lipid complex, and a medicinal composition comprising a pharmaceutical and the above protein-lipid complex.

The present invention will be described in detail, hereinafter.

The reception of tastes on the tongue is effected through specific mechanisms which correspond to particular flavours. More precisely, it is presumed that sweetness and amino-acidic tastes are perceived through specific receptor proteins, and that ion channels participate in the reception of salty and sour tastes. It is also known that bitterness is received by the lipid layer of the taste-receiving membrane.,

Generally, bitter-tasting substances have hydrophobic groups in order to exhibit high affinity for lipid membranes. This has been confirmed by the use of an artificial lipid membrane: when a bitter-tasting substance is added to a lipid membrane containing a fluorescent dye, the fluorescent dye is released. More fluorescent dye is released by the addition of a bitter-tasting substance having a stronger bitter taste. When another flavouring substance is added, no fluorescent dye is released. Accordingly, it is believed that the fluorescent dye is displaced by the bitter-tasting substance adsorbed by the lipid membrane. Thus, the bitter-tasting substance has the property of being easily adsorbable by a lipid membrane. Accordingly, it is reasonable to utilize lipid membrane in order to decrease bitterness.

However, the handling of lipid is difficult in practice. For example, when a neutral lipid such as a triglyceride or a phospholipid is used to decrease bitterness, an emulsification and dispersion step is necessary for in preparing a foodstuff or a pharmaceutical having a decreased bitter taste, which is practically disadvantageous.

On the contrary, the protein-lipid complex of the present invention can take the form of powder, fine particles or paste, so that it can be mixed to use as such with a food, a pharmaceutical or a cosmetic having a bitter taste, being easy to use in practice. Further, since a protein is bonded to a lipid in the protein-lipid complex of the present invention, the dispersibility of the lipid in water is improved. Accordingly, when the foodstuff, pharmaceuticals or cosmetic having a bitter taste is liquid, the complex can be suspended in it.

As described above, when the protein-lipid complex of the present invention is added to a foodstuff, pharmaceutical or cosmetic containing a bitter-tasting component, the bitterness is decreased by the following two effects:

1) when the protein-lipid complex of the present invention is held in the mouth, the complex is adsorbed in the neighbourhood of the bitterness receptors of the taste buds, so blocking the reception of bitter substances and

2) bitter-tasting substances are adsorbed by the lipid moiety of the protein-lipid complex of the present invention, thus lowering their concentration in the mouth.

Further, by virtue of the decrease in bitterness attained by the above mechanisms, sweetness and flavour can be sensed at an enhanced intensity by the taste receptors, which is presumably a reason that tastes can be modified by the use of the protein-lipid complex according to the present invention.

The protein-lipid complex according to the present invention will be described in detail hereinafter.

The proteins which can be used in preparing the protein-lipid complex according to the present invention include animal proteins and/or plant proteins. Specific examples thereof include milk protein, soybean protein, egg protein and wheat protein. In the present invention, it is preferable to use a water-soluble protein, particularly one or more of β-lactoglobulin, -lactalbumin, casein, serum albumin, ovalbumin, glycinin, actin and myosin. It is most desirable to use a protein (mixture) comprising at least 30% by weight of β-lactoglobulin.

Lipids which can be used in preparing the protein-lipid complex according to the present invention include monoglycerides, diglycerides, triglycerides, phospholipids, lysophospholipids, glycolipids, sterol lipids, fatty acid esters of polyols, and fatty acids. Among them, phospholipids and lysophospholipids are preferred, and phospholipids are particularly preferable. Further, specific examples of phospholipids include phosphatidylcholine, phosphatidylethanolamine, phosphatidylinositol, phosphatidic acid, phosphatidylserine, phosphatidylglycerol, cariolipin and sphingomyelin, while those of lysophospholipids include lysophosphatidylcholine and lysophosphatidic acid. Furthermore, as the above phospholipid and lysophospholipid, processed lecithins prepared by subjecting lecithin and/or lecithin analogues originating from animals and/or plants to enzymatic treatment and/or enzymolysis can be used. In addition, as the above phospholipid and lysophospholipid, not only those occurring naturally, but also those prepared by chemical or enzymatic synthesis can be used. Specific examples of such phospholipid and lysophospholipid include reaction products prepared by a synthesis method such as esterification of a diglyceride with phosphoric acid, that of a monoglyceride with phosphoric acid and that of glycerophosphoric acid with a fatty acid. Such reaction products include monoacyl glyceromonophosphates, monoacyl glycerodiphosphates, diacyl glyceromonophosphates, bisphosphatidic acid and so forth. Further, examples of the synthetic phospholipid and lysophospholipid include hydrogenated phospholipids and hydrogenated lysophospholipids. Among these lipids, negatively charged acidic phospholipid is particularly preferable. More specifically, preferable examples thereof include phosphatidylinositol, phosphatidic acid, phosphatidylserine, phosphatidylglycerol and cardiolipin.

The protein-lipid complex of the present invention can be prepared as a powdery, particulate, granular, or pasty composition by homogeneously dispersing a protein, a lipid and, if necessary, other components in water by mechanical means and/or sonication and then effecting dehydration.

When the protein-lipid complex of the present invention is used for the purpose of decreasing the bitterness of a pharmaceuticals the ratio between the protein incorporated and the lipid incorporated in the preparation of the protein-lipid complex is preferably such that the lipid content is from 0.01 to 100 parts (weight ratio) based on 1 part of the protein, and is still more preferably one that the lipid content is from 1 to 10 parts (weight ratio) based on 1 part of the protein.

The protein-lipid complex of the present invention can be prepared by using water in an amount of preferably from 0.01 to 100 parts (weight ratio), still more preferably from 1 to 10 parts (weight ratio), based on 1 part of the total amount of the protein and the lipid which have been blended at the ratio described above, mixing them, dispersing and emulsifying by mechanical means and/or sonication, and dehydrating the obtained emulsion composition.

When the protein-lipid complex of the present invention is used to modify the taste of a food or of a cosmetic (including decreasing the bitterness thereof), the ratio between the protein incorporated and the lipid incorporated in the preparation of the protein-lipid complex is preferably such that the lipid content is from 0.5 to 50 parts by weight based on 1 part of the protein, and is still more preferably one that the lipid is from 0.4 to 3 parts by weight ratio to 1 part of the protein.

In the preparation of an emulsion by mixing a protein with a lipid in the ratio described above and dispersing the resulting mixture in water or by adding a protein and a lipid to water separately and dispersing them, it is preferably to use water in an amount of from 0.5 to 100 parts (weight ratio), still more preferably from 1 to 10 parts (weight ratio) based on 1 part of the total amount of the protein and lipid.

The emulsification step for preparing the protein-lipid complex according to the present invention can be conducted for example by a method which comprises premixing the protein with the lipid and dispersing and emulsifying the resulting mixture in a predetermined amount of water, or a method which comprises preparing a homogeneous aqueous solution of the protein and dispersing and emulsifying the lipid in the solution. Other methods may also be used. In the dispersion and emulsifying step, a homogenizer or an emulsifier or an ultrasonic apparatus can be used.

With respect to the emulsifying condition of the emulsion composition, oil-in-water type (O/W type), water-in-oil type (W/O type), multiphase emulsion types such as oil-in-water-in-oil type (O/W/O type), and so forth may be cited. The conditions for emulsifying are not particularly critical. It is preferable that the particle size of the dispersed phase of the emulsion composition be 0.1 to 100 μm, especially 0.5 to 10 μm.

Although the temperature at which the blending of the protein with the lipid or the dispersion of a protein and a lipid in water is conduted is not very critical, operation at high temperature causes the degradation of the lipid and the generation of a bad smell in some cases. Accordingly, it is preferable that the temperature be 60°C or below to prevent such adverse effects. The method of dehydration is not particularly limited, and known methods can be employed. Examples of such methods include means such as vacuum drying, spray drying and freeze drying. It is desirable in the present invention to employ a method which permits rapid dehydration without causing the degradation of the lipid and protein and the contamination with microorganisms.

The form of the protein-lipid complex depends upon the water content and the lipid content. The complex generally contains water in an amount of 20% by weight or below, preferably 12% by weight or below, still more preferably 10% by weight or below.

Next, the method for modifying the taste of a foodstuff, pharmaceutical or cosmetic with the use of the above protein-lipid complex and the method for decreasing bitterness in a foodstuff, pharmaceutical or cosmetic therewith will be described in detail.

When the pharmaceutical having a bitter- tasting component is in the form of a liquid or an aqueous solution, the above protein-lipid complex may be added to the liquid or the aqueous solution in a final concentration of 0.01 to 99% (weight ratio), preferably 0.1 to 20% (weight ratio) and thereafter the obtained mixture may be sufficiently agitated to disperse the complex in the liquid. In the stirring and dispersion steps, a homogenizer, an emulsifier and an ultrasonic apparatus may be used, for example, Further, the resulting stirred dispersion may be converted into a solid such as powder by subjecting it to dehydration.

When the pharmaceutical having a bitter tasting component is in the form of a paste or a solid, the protein-lipid may be added to the paste or the solid in an amount of at least 0.1 part (weight ratio), preferably 10 to 500 parts (weight ratio) base on 1 part of the paste or the solid, and the obtained mixture homogenized.

When the protein-lipid complex of the present invention is used to modify the taste of food, it is preferable to use the complex in an amount of 0.05 to 10% by weight, still more preferably 0.1 to 3.0% by weight based on the total amount (i.e., 100% by weight) of the food containing the complex.

When the above protein-lipid complex is used for decreasing the bitterness of a cosmetic, it is preferable to incorporate the complex in the cosmetic in a concentration of 0.05 to 10% by weight, particularly 0.1 to 5% by weight. Further, it is preferable to use the complex in an amount of 1 to 1000 times, particularly 5 to 200 times the weight of the bitter-tasting cosmetic component.

When the complex of the present invention is used for the purpose of decreasing the bitterness of a food, pharmaceutical or cosmetic containing a bitter-tasting component which hardly soluble in water, an organic solvent, e.g., an alcohol such as ethanol or a hydrocarbon such as hexane is used, the food is dissolved therein, and then the protein-lipid complex is added thereto.

Foodstuffs, to which the taste-modifying agent, taste-modifying method, bitterness-decreasing agent or bitterness-decreasing method according to the present invention can be applied, including citrus fruits, e.g. grapefruit, orange or lemon, and juices thereof; other fruits and vegetables, such as tomato, green pepper, celery, gourd, carrot, potato or asparagus and juices thereof; seasonings such as sauce, soya and miso (fermented soya); soybean foods such as tofu and soybean milk; emulsion foods such as cream sauces, dressings, mayonnaise and margarine; processed marine products such as fish meat, ground fish and fish eggs; legumes such as peanuts; luxury goods such as beer, coffee, green tea, fermented teas, e.g. black tea, and cocoa; bread; pickles; chewing gum; confectionery; snack foods; cheese, peppermint; soft drinks; soups such as powdered soup; dairy goods; powdered dairy drinks and pasta/noodles.

Further, the complex of the present invention can be used for decreasing the bitterness of bitter-tasting amino acids such as leucine, isoleucine and phenylalanine, and peptides and oligosaccharides which originate from food.

The complex of the present invention can also be used for decreasing the bitternesses of potassium chloride or magnesium chloride which are used as substitutes for common salt.

Most of foods contain a bitter-tasting substance. However, they are generally eaten without sensing the bitterness by virtue of the influence of other tasting components. The protein-lipid complex of the present invention can decrease the influence of the bitter-tasting components in foods, thus modifying the overall flavour thereof.

Pharmaceuticals, to which the taste-modifying agent, taste-modifying method, bitterness-decreasing agent and bitterness-decreasing method according to the present invention can be applied, include bitter-tasting drugs used as medicines. In particular, the complex of the present invention is preferably used for decreasing the bitterness of an acid-addition salt of a basic pharmaceutical. Specific examples thereof include mineral acid salts such as hydrochlorides, nitrates and sulfates) of basic pharmaceuticals; and, further, organic acid salts (such as acetate, citrate, tartrate, maleate, lactate, carbonate, hydrogen-carbonate and borate salts), of basic pharmaceuticals.

The forms of the bitter-tasting pharmaceuticals themselves or the preparations containing the pharmaceuticals include hydrolyzate (aqueous solution), suspension, emulsion and solid.

If necessary, one or more additives may further be added to the pharmaceutical preparation, i.e., a medicinal composition, which contains the bitterness-decreasing agent according to the present invention. Specific examples of such additives include fillers, binders, disintegrators, lubricants, fluidizing agents, coating agents, corrigents, masking agents, flavouring agents, antioxidants, and mixtures thereof.

The granulator used in the preparation step of the pharmaceutical preparation may for example be a planetary mixer, an agitation granulator, a high-speed mixing granulator, an extruding granulator, a fluidized-bed granulator, a centrifugal tumbling granulator, or a roller compactor.

Examples of the final dosage form of such pharmaceutical preparations includes capsules, granules, pills, suspensions, emulsions, powders, tablets, infusions, decoctions and troches, as well as liquid forms such as extracts, elixirs, spirits, syrups, aromatic waters, lemonades and liquid extracts.

Cosmetics, to which the taste-modifying agent, taste-modifying method, bitterness-decreasing agent and bitterness-decreasing method according to the present invention can be applied, include those used for the face and those used for the oral cavity. Specific examples or the cosmetics for the face include skin lotions, milky lotions, creams, face packs, lipsticks, foundations, shaving preparations, after-shave lotions, cleansing foams and cleansing gels. Cosmetics for the oral cavity include dentifrices, mouthwashes, mouthrinses.

The bitter-tasting components present among the raw materials for these cosmetics include surfactants such as sodium alkyl sulfate and sodium monoalkyl phosphate; fragrances such as menthol, linalool, phenylethyl alcohol, ethyl propionate, geraniol, linalyl acetate and benzyl acetate; antimicrobials such as methyl paraben, propyl paraben and butyl paraben; humectants such as lactic acid and sodium lactate; alcohol-denaturating agents such as sucrose octaacetate and brucine; and astringents such as aluminium lactate.

These cosmetic components are generally contained in a cosmetic in an amount of 0.00001 to 5% by weight.

As described above, the protein-lipid complex of the present invention can be used mixed with a foodstuff, pharmaceutical or cosmetic, in other words, as a component of a food composition, medicinal composition or cosmetic composition. Alternatively, the complex may be used in a manner which comprises holding or chewing the protein-lipid complex in the mouth and then taking the foodstuff, pharmaceutical or cosmetic containing bitter-tasting component into the mouth. In this case, the bitterness-decreasing mechanism is one wherein the protein-lipid complex blocks the bitterness-receptor sites present in the oral cavity. The protein-lipid complex to be held in the oral cavity in this case may be any of a solid and an aqueous dispersion.

More specifically, the protein-lipid complex may be in the form of particles such as granules or fine granules. Such a preparation may be added to water in a predetermined amount to suspend or dissolve; and then the suspension or solution thus obtained may be held in the oral cavity in order to block the bitterness-receptor sites, so that a bitter taste is not sensed on subsequent oral ingestion of a bitter-tasting pharmaceutical. The dosage form of the complex to be used as a suspension or solution may include tablets, effervescent powders, and capsules, in addition to granules and fine granules. Alternatively, a liquid preparation such as a syrup or an emulsion, or an aerosol or other spray may be prepared by suspending or dissolving the protein-lipid complex in water and the resulting product may be held in the oral cavity prior to the ingestion of a bitter-tasting foodstuff, pharmaceutical or cosmetic.

The bitterness-receptor sites can also be blocked also preparing a pharmaceutical, food or drink, which contains the protein-lipid complex of the present invention, such as troche, sweet and chewing gum and holding the pharmaceutical, food or drink in the oral cavity before and/or simultaneously with the oral ingestion of a bitter-tasting food, pharmaceutical or cosmetic.

In the accompanying drawings:

Fig. 1 is a bar graph showing the sweetness-increasing effect of one protein-lipid complex of the present invention.
Fig. 2 is a bar graph showing the umami-increasing effect of one protein-lipid complex of the present invention.
Fig. 3 is a bar graph showing the bitterness-depressing effect of one protein-lipid complex of the present invention.
Fig. 4 is a bar graph showing the influence of one protein-lipid complex of the present invention upon sourness.
Fig. 5 is a bar graph showing the influence of one protein-lipid complex of the present invention upon saltiness.
Fig. 6 is a bar graph showing the appearance of the influence of the protein-lipid complex of the present invention on the change in membrane potential caused by quinine sulfate.
Fig. 7 is a graph showing the relationship between the amount of one protein-lipid complex of the present invention added and the response of the chorda tympani nerve system of a rat to stimulation when the complex is added to quinine sulfate.
Fig. 8 is a graph showing the relationship between the amount of one protein-lipid complex of the present invention added and the response of the chorda tympani nerve system of a rat to stimulation when the complex is added to sucrose.
Fig. 9 is a bar graph showing the depression effects of one protein-lipid complex of the present invention upon the bitternesses of quinine, strychnine and papaverine.

Fig. 10 is a bar graph showing the depression effects of one protein-lipid complex of the present invention upon the bitternesses of caffeine and L-leucine.

Fig. 11 is a bar graph showing the depression effects of one protein-lipid complex of the present invention upon the bitternesses of caffeine and quinine.

The present invention will now be described in more detail with reference to the Examples:

Preparative Example 1

100 g of a powdery concentrate of whey protein (trade name: SANRAKUTO N-2, a product of Taiyo Kagaku Co., Ltd.) was kneaded with 40 g of soybean lecithin (trade name: Nissin DX, a product of The Nissin Oil Mills, Ltd.) and the obtained kneaded mixture was dispersed in 1 ℓ of water. Then, the obtained dispersion was agitated by the use of a TK homomixer mfd. by Tokushu Kika Kogyo Co., Ltd. at 9000 rpm for 15 minutes to conduct homogenization.

The obtained emulsion was vacuum-dehydrated at 40°C at 0.1 Torr to reduce the water content of the solid to 9.8%. The obtained massive composition was pulverized and passed through a 20-mesh sieve to give 136 g of a protein-lipid complex powder (Invention sample a).

Preparative Example 2

To the step of the preparation of the emulsion was effected in the same manner as that of the above Preparative Example 1. Then, such emulsion was dried by the use of a freeze dryer to reduce the water content of the solid to 7.8%. The obtained composition was passed through a 20-mesh sieve to give a protein-lipid complex powder (Invention sample b).

Preparative Example 3

100 g of a powdery concentrate of whey protein (trade name: SANRAKUTO N-5, a product of Taiyo Kagaku Co., Ltd.) was dissolved in 1 ℓ of distilled water. 20 g of soybean lecithin (a product of Ajinomoto Co., Ltd.) was added to the obtained aqueous solution in portions under agitating the aqueous solution with a TK homomixer mfd. by Tokushu Kika Kogyo Co., Ltd. at 9000 rpm to emulsify the soybean lecithin at room temperature. The obtained dispersion was freeze-dried to reduce the water content of the solid to 6.5%, giving 108 g of a solid product. This solid product was pulverized with a metal spatula to give a protein-lipid complex powder (Invention sample c).

Preparative Example 4

80 g of soybean lecithin (trade name: Epikuron 200, a product of Lucas Meyer) was dispersed in 1 ℓ of distilled water by sonication. 100 g of a powdery concentrate of milk whey (trade name: Milpro H, a product of San-Ei Kagaku) was added to the obtained dispersion, followed by aditation. The obtained emulsion was freeze-dried to reduce the water content of the solid to 6.8%. The resulting solid product was passed through a 20-mesh sieve to give 167 g of a protein-lipid complex powder (Invention sample d).

Preparative Example 5

80 g of soybean lecithin (trade name: Epikuron 200, a product of Lucas Meyer) was dispersed in 1 ℓ of distilled water by sonication. 40 g of a powdery concentrate of milky whey (trade name: Milpro H, a product of San-Ei Kagaku) and 60 g of sodium caseinate (a product of San-Ei Kagaku) were added to the obtained dispersion, followed by agitation. The obtained emulsion was freeze-dried until the water content of the solid was reduced to 6.8%. The resulting solid product was passed through a 20-mesh sieve to give 170 g of a protein-lipid complex powder (Invention sample e).

Preparative Example 6

80 g of soybean licithin (trade name: Epikuron 200, a product of Lucas Meyer) was dispersed in 1 ℓ of distilled water by sonication. 100 g of a skimmilk powder (a product of Snow Brani Milk Products Co., Ltd.) was added to the obtained dispersion, followed by agitation. The obtained emulsion was freeze-dried to reduce the water content of the solid to 6.5%. The resulting solid product was passed through a 20-mesh sieve to give 165 g of a protein-lipid complex powder (Invention sample f).

(Example 1)

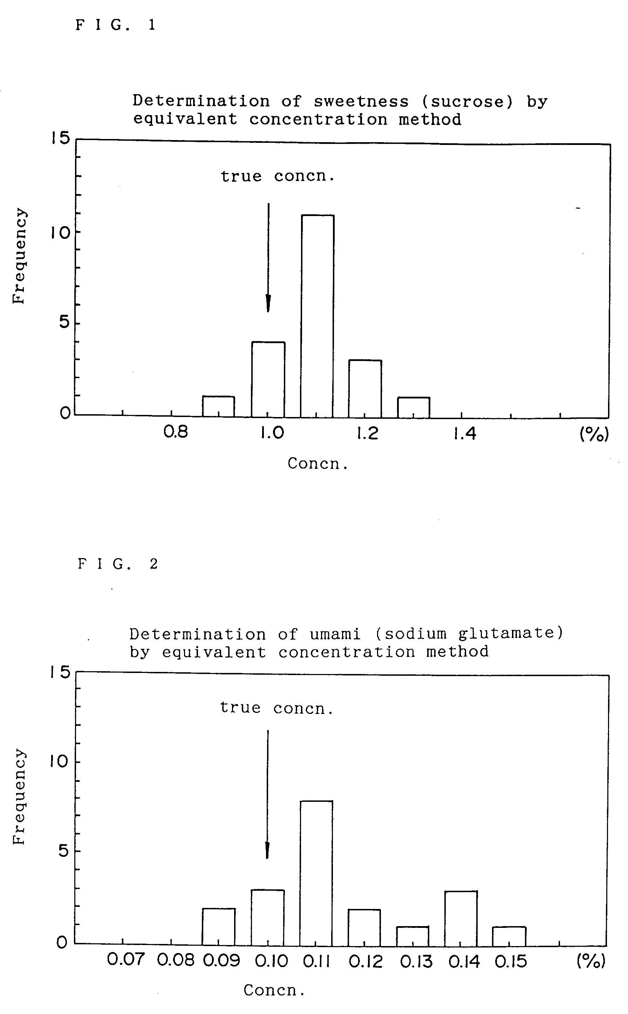

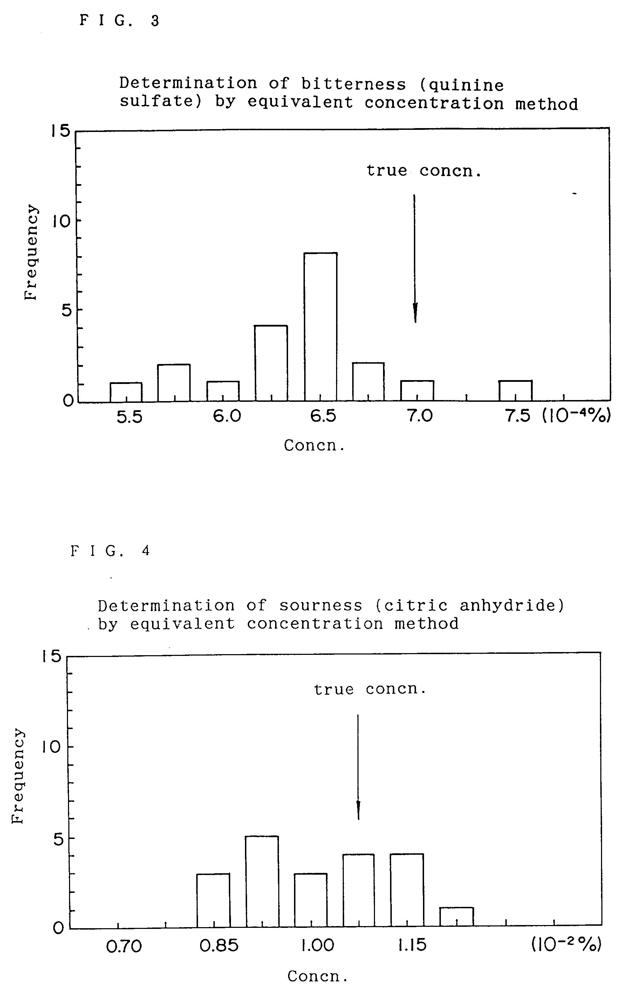

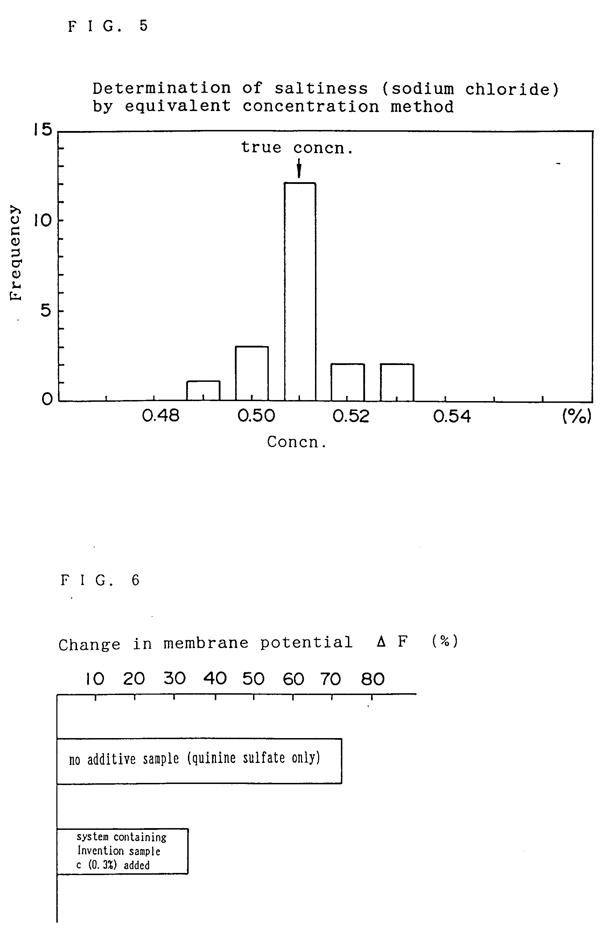

Invention sample a was tested for its taste-modification effects upon five basic tastes by the equivalent concentration method. The "equivalent concentration method" refers to a method which comprises comparing the intensity of taste of a test solution with those of several standard solutions to determine which standard solution was equivalent to the test solution in the intensity of taste. Solutions each containing a tasting substance in a concentration specified in Table 1 were prepared and Invention sample a was added thereto in a final concentration of 0.1 %. The results are given in Figs. 1 to 5. As shown in Figs. 1 and 2, the complex of the present invention exhibited an increasing effect upon sweetness and umami, while as shown in Fig. 3, it exhibited a depression effect upon bitterness. Further, as shown in Figs. 4 and 5, the complex exerted little influence upon sourness and saltiness.

Table 1

| Test tasting substances and concentrations | | |
|---|---|---|
| Kind of taste | Test tasting substance | Concn. of test soln. (%) |
| sweetness | sucrose | 1.0 |
| umami | sodium glutamate | 0.10 |
| bitterness | quinine sulfate | 0.00070 |
| sourness | citric anhydride | 0.01075 |
| saltiness | sodium chloride | 0.51 |

(Example 2)

Invention sample b was added to each test solution specified in the Table 1 in a final concentration of 0.3%. The obtained solutions were tested by the equivalent concentration method to examine the influences of the Invention sample b upon each taste. The results are given in Table 2. As is apparent from table 2, the complex of the present invention increased sweetness and umami, depressed bitterness, and had no influence upon sourness and saltiness.

Table 2

| Kind of taste | Concn. recognized as to test soln. containing Invention sample b (%) | True concn. of test soln. (%) |
|---|---|---|
| sweetness | 1.15 | 1.0 |
| umami | 0.13 | 0.10 |
| bitterness | 0.00060 | 0.00070 |
| sourness | 0.0101 | 0.01075 |
| saltiness | 0.51 | 0.51 |

(Example 3)

Invention sample a was added to aqueous solutions of sucrose having various concentrations ranging from 0 to 0.6% in a final concentration of 0.3% and the effect of Invention sample a upon sweetness was evaluated by determining the stimulus threshold. Each examinee held the aqueous solutions of sucrose having various concentration at random and selected the solutions in which he or she sensed sweetness. The one having the minimum concentration among them which was selected by the examinee was regarded as the threshold. The results are given in Table 3. As is understood from the results in Table 3, lowered thresholds were observed with respect to the systems containing Invention sample a, which reveals that the addition of the complex according to the present invention makes a person more sensitive to sweetness.

Table 3

| Examinee | | A | B | C | D | E | F | G | H | I | J | Average value |
|---|---|---|---|---|---|---|---|---|---|---|---|---|
| Sweetness threshold (%) | aq. sucrose soln. | 0.1 | 0.4 | 0.6 | 0.3 | 0.3 | 0.2 | 0.3 | 0.5 | 0.2 | 0.4 | 0.33 |
| | added 0.3% of Invention sample a | 0.1 | 0.1 | 0.2 | 0.3 | 0.2 | 0.1 | 0.1 | 0.2 | 0.1 | 0.1 | 0.15 |

(Example 4)

Invention sample b was added to a commercially available milk containing 1.875% of sucrose in a final concentration of 0.1%. In a similar manner to that of Example 1, the equivalent concentration method was effected with the use of milk containing sucrose in various concentrations as the standard solution. As the result, the recognized sucrose concentration was 2.25%, which means that the complex of the present invention exhibited the effect in milk.

(Example 5)

Invention sample c was added to a 1.0% aqueous solution of sucrose and a 0.00070% aqueous solution of quinine sulfate each in a final concentration of 0.1%. The resulting solutions were tested by the equivalent concentration method. As the result, the recognized concentrations were 1.25% and 0.00058%, respectively.

(Example 6)

Invention sample c was examined for taste-modifying effect by the use of a bitterness-responsive system using a model biomembrane. A single-lamellar liposome of azolectin was prepared according to the conventional process and the adsorption of quinine sulfate onto the liposome was determined. More precisely, the change in the intensity of fluorescence of a dyestuff sensitive to membrane potential, i.e., diS-$C_3$ (5) (a product of Japanese Research Institute for Photosensitizing Dyes Corporation) was determined by the method of Kumazawa et al. (Biochemistry, Vol. 27, p.1239, 1988) as an indication of the change in membrane potential due to the adsorption of quinine sulfate onto the membrane. The results are given in Fig. 6. It is presumed that the complex of the present invention acted on the membrane to depress bitterness.

(Example 7)

The capability of Invention sample c of adsorbing hydrophobic substances was determined by partitioning the Invnetion sample c in an organic solvent-water binary system (Yukagaku (Oil Chemistry), Vol. 30, No. 11, p.942). More precisely, the experimentation was effected as follows: Invention sample c was added to 30 ml of a 0.02 M pottasium phosphate buffer (pH : 7.0) in a concentration of 3%. 10 ml of a 0.1% solution in n-heptane of n-octanol, n-decanol, n-dodecanol, δ-decalactone or δ-dodecalactone was superposed on the mixture thus obtained. The aqueous phase was mildly stirred with a magnetic stirrer and after 6 hours, the concentration of n-octanol, n-decanol, n-dodecanol, δ-decalactone or δ-dodecalactone in the n-heptane phase was determined by gas chromatography. The one wherein no sample was added to the aqueous phase was prepared as a control and the same procedure as described above was repeated to determine the concentrations. When the concentrations of n-octanol or the like in the n-heptane phase observed with respect to the control and those of n-octanol or the like in the n-heptane phase observed with respect to the system containing Invention sample c were abbreviated to "$C_n$" and "$C_s$", respectively, the amounts of n-octanol or the like partitioned to the aqueoues phases are represented by $W_n = \{1-(C_n/0.1)\}$ and $W_s = \{1-(C_s/0.1)\}$, respectively. Accordingly, the amount of n-octanol or the like adsorbed onto the Invention sample c is represented by the difference between the amounts thereof partitioned to the aqueous phases, i.e., $(W_s - W_n) \times 100$ (%). The results are given in Table 4.

Table 4

| Adsorbate | Adsorbed amount |
|---|---|
| n-octanol | 19.7 |
| n-decanol | 19.0 |
| n-dodecanol | 15.6 |
| δ-decalactone | 15.0 |
| δ-dodecalactone | 16.2 |

(Example 8)

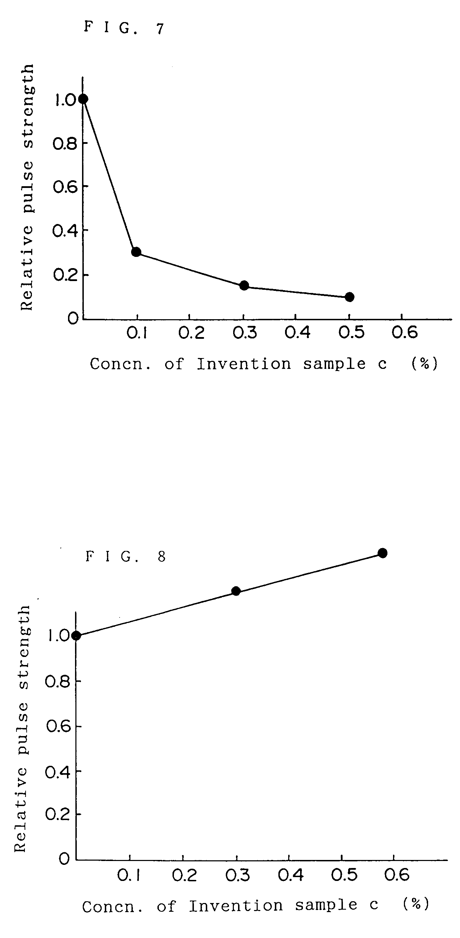

The gustatory responses of Invention sample c upon sweetness and bitterness were determined based on the response of the chorda tympani nerve system of a rat to stimulation. The lower jaw of a rat was cut to expose the funicular nerve. After a silver electric lump had been brought into contact with the funicular nerve, the electrical pulse generated by gustatory stimulation was conducted to an amplifier and recorded. 0.5 mM aqueous solutions of quinine sulfate or 1 M aqueous solutions of sucrose containing Invention sample c in concentrations ranging from 0 to 0.5% were each circulated in the oral cavity of the rat to record the response potential. The results are given in Figs. 7 and 8, respectively. It can be understood from the Fig. 7 that the stimulation by bitterness is decreased by the addition of the complex of the present invention, while it can be understood from the Fig. 8 that the stimulation by sweetness is increased thereby. Accordingly, it can be understood that the protein-lipid complex of the present invention acts at the taste receiving level.

(Example 9)

According to the following formulation, preparation process and evaluation method, a whipped cream and other whipped cream not containing Invention sample d were prepared and compared with each other in creamy flavor.

〔Formulation〕

| (oil phase) | |
|---|---|
| • hardened rapeseed oil (m.p.: 32.8°C) | 20 pt. (by wt.) |
| • hardened coconut oil (m.p.: 34.5°C) | 6 |
| • butter fat (m.p.: 32.4° C) | 14 |
| • lecithin | 0.28 |
| (aqueous phase) | |
| • water | 54.5 |
| • skimmilk powder | 4 |
| • fatty acid ester of sucrose (HLB 11) | 0.2 |
| • hexametaphosphoric acid | 0.1 |
| • Invention sample d | 0.5 |

〔Preparation process〕

An oil phase and an aqueous phase were each prepared by dissolving or dispersing the raw materials in each other. According to the conventional process, a creamy fat-and-oil composition was prepared with the oil phase and the aqueous phase thus obtained through preliminary emulsification, high-pressure homogenization and sterilization. 80 g of granulated sugar was added to 1 ℓ of the composition and the obtained mixture was whipped to give a whipped cream.

〈Evaluation〉

The whipped cream containing Invention sample d was organoleptically examined for creamy flavor by taking, as a control, a whipped cream not containing Invention sample d. The results are given in Table 5. The figures in the Table 5 indicate each an average of the points graded by twenty expert panelists, in which a case wherein the control is superior to the sample is ranked as "-1"; a case wherein the control is slightly superior to the sample is ranked as "-0.5"; a case wherein the sample is equivalent to the control as "0"; a case wherein the sample is slightly superior to the control as "+0.5" and a case wherein the sample is superior to the control as "+1". As is apparent form Table 5, the product containing the complex of the present invention was such a cream that it exhibited a stronger milky flavor and a better body and was free from the disagreeable tastes such as bitterness and astringent taste, as compared with the product not containing the complex.

Table 5

| Evaluation item | whole | milkiness | body | sweetness | disagreeable taste |
|---|---|---|---|---|---|
| Point | 0.8 | 0.6 | 0.7 | 0.55 | -0.4 |

(Example 10)

The same procedure as that of Example 9 was repeated except that Invention sample e was used instead of Invention sample d. The results are given in Table 6. By adding the complex of the present invention, the whipped cream was improved in flavor, as compared with the control.

Table 6

| Evaluation item | whole | milkiness | body | sweetness | disagreeable taste |
|---|---|---|---|---|---|
| Point | 0.6 | 0.5 | 0.5 | 0.4 | -0.4 |

(Example 11)

Invention samples d and e were each treated with SDS (sodium dodecyl sulfate) and mercaptan, and then were each subjected to SDS-polyacrylamide gel electrophoresis. The result of the electrophoresis was analyzed with a densitometer to determine the amounts of proteins. The results are given in Table 7. It is preferable for preparing a product excellent in flavor that the $\beta$-lactoglobulin content be 30% or above.

Table 7

| Protein | Sample | |
|---|---|---|
| | Invention sample d | Invention sample e |
| $\beta$-lactoglobulin | 80% | 32 |
| $\alpha$-lactalbumin | 16% | 9 |
| $\alpha$-casein | 0% | 43 |
| serum albumin | 2% | 5 |
| others | 2% | 11 |

(Example 12)

1 ℓ of purified water was added to a mixture comprising 100 g of acetaminophen (acetamidophenol) and 60 g of Invention sample d, followed by mixing. 150 g of synthetic aluminum silicate and mannitol were added to the mixture. The obtained mixture was further kneaded and vacuum-dried to give a powder. As compared with original acetaminophen powder, the obtained powder little tasted bitter.

(Example 13)

Each of the bitter-tasting cosmetic components listed in Table 8 and the protein-lipid complex prepared in the above Preparative Example 1 (Invention sample a) were dissolved or dispersed in water in predetermined concentrations (see Table 8), and the obtained solution or dispersion was evaluated for bitterness according to the following criteria. The results are given in Table 8.

| | |
|---|---|
| no bitter taste | 0 |
| slightly bitter taste | 1 |
| distinctly bitter taste | 2 |
| strongly bitter taste | 3 |
| severely bitter taste | 4 |
| severely bitter taste which remains even after rinse | 5 |

Table 8

| Cosmetic component | | Concn. of protein-lipid complex | | | |
|---|---|---|---|---|---|
| Compd. | concn. (wt%) | 0 wt% | 1.0 wt% | 3.0 wt% | 5.0 wt% |
| L-menthol | 0.10 | 3 | 2 | 2 | 1 |
| sodium monocetyl phosphate | 0.40 | 3 | 2 | 1 | 0 |
| sodium lauryl sulfate | 0.50 | 3 | 2 | 1 | 0 |
| linalol | 0.10 | 2 | 1 | 1 | 0 |
| phenylethyl alcohol | 0.10 | 2 | 1 | 0 | 0 |
| sucrose octaacetate | 0.01 | 5 | 4 | 3 | 2 |

(Example 14)

A dentifrice comprising the following components was prepared in the conventional manner:

| | |
|---|---|
| • calcium secondary phosphate dihydrate | 45.0% |
| • silicic acid anhydride | 2.0% |
| • sorbitol | 15.0% |
| • carboxymethylcellulose | 1.5% |
| • sodium monolauryl phosphate | 2.0% |
| • flavoring material | a proper amount |
| • protein-lipid complex (Invention sample a) | 3.0% |
| • water | the balance |

This dentifrice was one decreased in the bitterness due to sodium monolauryl phosphate.

(Example 15)

A mouthwash having the following composition was prepared in the conventional manner:

| | |
|---|---|
| • ethanol | 15.0% |
| • sorbitol | 10.0% |
| • saccharin sodium | 0.15% |
| • L-menthol | 0.10% |
| • sodium lauryl sulfate | 0.10% |
| • protein-lipid complex (Invention sample b) | 1.0% |
| • water | the balance |

This mouthwash was one decreased in the bitterness due to L-menthol and sodium lauryl sulfate.

(Example 16)

A skin lotion having the following composition was prepared in the conventional manner:

| | |
|---|---|
| • glycerol | 5.0% |
| • 1,3-butanediol | 5.0% |
| • denatured ethanol (containing 0.1% of sucrose octaacetate) | 10.0% |
| • polyoxyethylene (20) octyl dodecyl ether | 1.0% |
| • fragrance | a proper amount |
| • protein-lipid complex (Invention sample c) | 0.5% |
| • methyl paraben | 0.1% |
| • water | the balance |

This skin lotion was one decreased in the bitterness due to sucrose octaacetate, so that the user was not displeased at the presence of the lotion remaining around the mouth after the application thereof.

(Example 17)

A milky lotion having the following composition was prepared in the conventional manner:

| | |
|---|---|
| • cyclic silicone (pentamer) | 20.0% |
| • squalane | 5.0% |
| • polyoxyethylene-modified silicone | 5.0% |
| • sodium lactate | 4.0% |
| • methyl paraben | 0.1% |

(continued)

| | |
|---|---|
| • fragrance | a proper amount |
| • protein-lipid complex (Invention sample d) | 2.0% |
| • water | the balance |

This milky lotion was one decreased in the bitterness due to sodium lactate, so that the user was not displeased at the bitterness even when the milky lotion was applied to the circumference of the mouth.

(Example 18)

A face cleansing preparation having the following composition was prepared:

| | |
|---|---|
| • triethanolamine monolauryl phosphate | 20.0% |
| • laurylbetaine | 5.0% |
| • glycerol | 5.0% |
| • polyoxyethylene (100) oleyl ether | 2.0% |
| • protein-lipid complex (Invention sample e) | 5.0% |
| • water | the balance |

This face cleansing preparation exhibited little bitterness even when it accidentally enters the mouth in washing the face.

(Example 19)

A mouth freshener having the following composition was prepared:

| | |
|---|---|
| • ethanol | 35.0% |
| • glycerol | 10.0% |
| • polyoxyethylene hardened castor oil | 1.0% |
| • L-menthol | 0.5% |
| • chlorohexidine gluconate | 0.02% |
| • protein-lipid complex (Invention sample f) | 1.0% |
| • water | the balance |

This mouth freshener little tasted bitter in spite of its high L-menthol content, and exhibited a high refreshing effect.

(Example 20)

10 g of a whey protein comprising β-lactoglobulin, lactalbumin and casein was kneaded with 8 g of soybean lecithin (trade name: SLP White, a product of True Lecithin Mfg. Co., Ltd.). The obtained kneaded mixture was dispersed in 50 ml of water and the obtained dispersion was homogenized with a homogenizer. The obtained emulsion was dehydrated under the conditions of 45°C and 0.1 Torr one whole day and night to give 15 g of a whey protein-lecithin complex having a water content of 8.5% by weight

(Invention sample 1).

This complex was evaluated for the decompression effect on the bitterness of pharmaceuticals.

The bitterness-decreasing effect can quantitatively be evaluated by determining the response of the glossopharyngeal nerve of a frog to the stimulation of the lingual surface thereof. The response of the gustatory organ of a frog to bitterness has a characteristic in that the bitterness threshold highly correlates with the results of human organoleptic test. Accordingly, the response of the gustatory organ of a frog to bitterness is usable as a system for the quantitative evaluation of the bitterness-decreasing effect.

The outside of the lower jaw of a frog was cut under urethane anesthesia to expose the glossopharyngeal nerve. The glossopharyngeal nerve was cut on its center side and brought into contact with a silver-silver chloride electrode on its periphery side. The electrical pulse (nerve impulse) caused by the stimulation by bitterness was amplified and integrated and thereafter recorded on a pen recorder. The height of the response observed just after the stimulation was taken as the response intensity to bitterness. The stimulation of the lingual surface was conducted by using the test liquids which will be described below each in a total amount of 10 ml at a flow rate of 1.5 ml/sec.

The complex of the present invention was examined for bitterness-decreasing effects on quinine hydrochloride, strychynine nitrate and papaverine as bitter-testing pharmaceuticals.

[Test liquids]

(1) 0.1 mM aqueous solution of quinine hydrochloride.
(2) a liquid prepared by adding the Invention sample 1 to a 0.1 mM aqueous solution of quinine hydrochloride in a final concentration of 0.5% by weight, stirring and mixing.
(3) 1 mM aqueous solution of strychnine nitrate.
(4) a liquid prepared by adding the Invention sample 1 to a 1 mM aqueous solution of strychnine nitrate in a final concentration of 0.5% by weight, stirring and mixing.
(5) 10 mM aqueous solution of papaverine.
(6) a liquid prepared by adding the Invention sample 1 to a 10 mM aqueous solution of papaverine in a final concentration of 0.5% by weight, stirring and mixing.

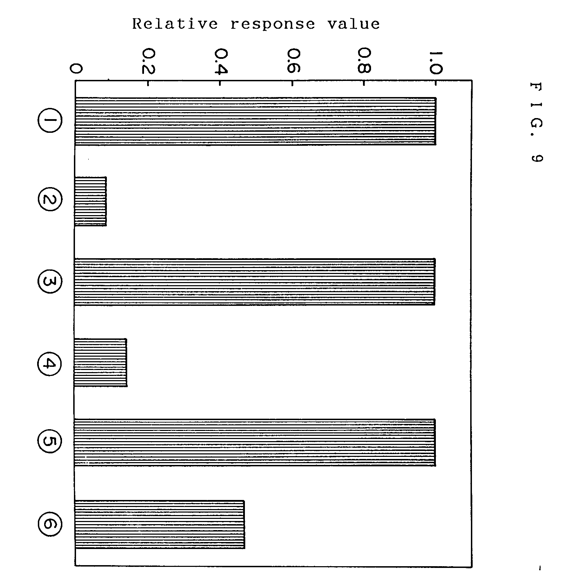

The results are given in Fig. 9.

(2); (4) and (6) are Examples, and (1), (3) and (5) are controls corresponding thereto, respectively.

As shown in the Fig. 9, the responses to the bitterness of these bitter-tasting pharmaceuticals were all decreased by the addition of the complex of the present invention.

(Example 21)

10 g of β-lactoglobulin was kneaded with 8 g of phosphatidic acid and the obtained kneaded mixture was dispersed in 50 ml of water. The obtained dispersion was homogenized with a homogenizer. The obtained emulsion was dehydrated under the conditions of 45°C and 0.1 Torr one whole day and night to give 15 g of a β-lactoglobulin-phosphatidic acid complex having a water content of 11.2% by weight (Invention sample 2).

The Invention sample 2 was evaluated for the bitterness-decreasing effects on caffeine and L-leucine as bitter-tasting substances in pharmaceuticals by determining the response of the glossopharyngeal nerve of a frog.

After a 0.3% dispersion of the Invention sample 2 was preliminarily held in the mouth of a frog for 10 minutes, an aqueous solution of caffeine or L-leucine was brought into contact with the lingual surface to stimulate the tongue. After water was held in the mouth of a frog for 10 minutes as a control, the tongue was stimulted with an aqueous solution of caffeine or L-leucine to determine the response of the glossopharyngeal nerve of a frog.

[Determination item]

(1) response to the stimulation by a 40 mM aqueous solution of caffeine after holding water for 10 minutes.
(2) response to the stimulation by a 40 mM aqueous solution of caffeine after holding a 0.3% dispersion of Invention sample 2 for 10 minutes.
(3) response to the stimulation by a 100 mM aqueous solution of L-leucine after holding water for 10 minutes.
(4) response to the stimulation by a 100 mM aqueous solution of L-leucine after holding a 0.3% aqueous dispersion

**15**

of Invention sample 2 for 10 minutes.

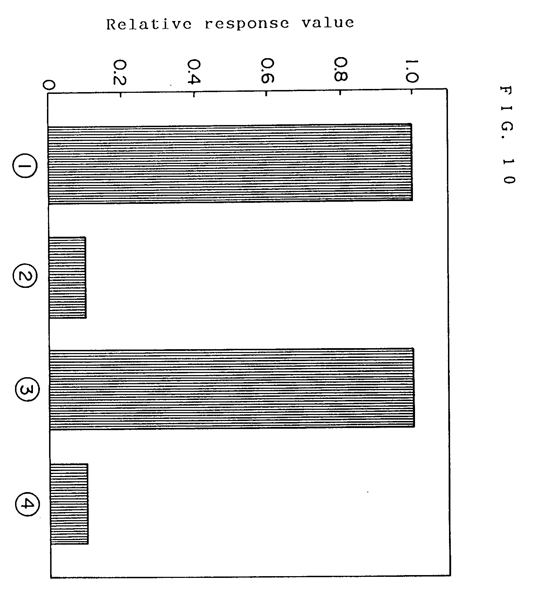

The results are given in Fig. 10.

As is apparent from Fig.10, every response to bitterness was decreased by pretreating the mouth of the frog with a 0.3% dispersion of the Invention sample 2.

(Example 22)

Invention sample 2 was added to a 1 mM aqueous solution of strychnine nitrate in a final concentration of 0.5% by weight. The obtained mixture was stirred to give a suspension. This suspension and a 1 mM aqueous solution of strychnine nitrate as a control were organoleptically evaluated for bitterness by twenty panelists for gustatory evaluation according to the following criteria:

| very strongly bitter | 5 |
|---|---|
| strongly bitter | 4 |
| bitter | 3 |
| slightly bitter | 2 |
| not bitter | 1 |

The results are given in Table 9. In Table 9, the average values of the grades obtained by the above organoleptic evaluation are shown. As is apparent from Table 9, the bitterness of strychnine nitrate was decreased by the addition of Invention sample 2.

Table 9

| Test sample | Organoleptic evaluation of bitterness (average value) |
|---|---|
| 1 mM aqueous solution of strychnine nitrate | 4.8 |
| added 0.5% by weight of Invention sample 2 to the above aqueous solution | 1.4 |

(Example 23)

0.1 g of crude caffeine powder was dissolved in 50 ml of deionized water, followed by the addition of 10 g of β-lactoglobulin and 8 g of phosphatidylcholine and stirring. The obtained mixture was homogenized with a homogenizer. The obtained emulsion was dehydrated by the use of a freeze dryer to give 16 g of a caffeine powder containing the bitterness-masking agent of the present invention, i.e., the protein-lipid complex. This caffeine powder little tasted bitter.

(Example 24)

100 g of ovalbumin originating in egg white was kneaded with 40 g of a lecithin prepared by enzymolysis and containing phosphatidylinositol and phosphatidic acid. 1000 ml of water was added to the obtained kneaded mixture. The obtained mixture was emulsified with a homomixer until being homogeneous. The obtained emulsion was dehydrated under the condition of 0.1 Torr one whole day and night to give 125 g of an ovalbumin-lecithin (prepared by enzymolysis) complex (Invention sample 3).

Invention sample 3 was added to grapefruit juice in final concentrations of 0.1 and 0.3% (weight ratio) to determine whether Invention sample 3 has the bitterness-decreasing effect to the grapefruit juice or not.

The grapefruit juices containing Invention sample 3 and one not containing it were examined for the intensity of the bitterness according to the five criteria as will be described below. Ten person of both sexes aged from 20 to 46 and having a nomal sensitivity for taste were selected as panelists.

| Intensity of bitterness | 5 | strongly bitter |
|---|---|---|
| | 4 | bitter |
| | 3 | somewhat bitter |
| | 2 | sensibly bitter |
| | 1 | not bitter |

The results are given in Table 10. The figures in Table 10 are the average values of the grades evaluated.

Table 10

| | Test sample | Concn. of Invention sample 3 added | Average value of intensity of bitterness |
|---|---|---|---|
| (1) | grapefruit juice | - | 4.2 |
| (2) | one obtained by adding Invention sample 3 to (1) | 0.1% | 1.4 |
| (3) | one obtained by adding Invention sample 3 to (1) | 0.3% | 1.0 |

(Example 25)

10 g of whey protein was kneaded with 4 g of an acidic phospholipid mixture comprising phosphatidylinositol, phosphatidylserine, phosphatidylglycerol and phosphatidic acid, and 100 ml of water was added to the obtained kneaded mixture. The obtained mixture was dispersed and homogenized by treating with a homogenizer. The obtained emulsion was dehydrated under the condition of 0.1 Torr one whole day and night to give 14 g of a whey protein-acidic phospholipid complex (Invention sample 4). Invnention sample 4 was added to coffee to determine whether Invention sample 4 exhibits the decreasing effect of the bitterness of coffee or not. Invention sample 4 was added to coffee in final concentrations of 0.1 and 0.3%. The intensity of the bitterness was evaluated according to the same five criteria as those of the Example 24. The results are given in Table 11.

Table 11

| | Test sample | Concn. of Invention sample 4 added | Average value of intensity of bitterness |
|---|---|---|---|
| (1) | coffee | - | 4.1 |
| (2) | one obtained by adding Invention sample 4 to (1) | 0.1% | 2.4 |
| (3) | one obtained by adding Invention sample 4 to (1) | 0.3% | 1.6 |

(Example 26)

100 g of a whey protein comprising β-lactoglobulin, α-lactalbumin and casein was kneaded with 40 g of soybean lecithin and the obtained kneaded mixture was dispersed in 1000 ml of water. The obtained dispersion was homogenized with a homogenizer. The obtained emulsion was freeze-dried under the conditions of 20°C and 0.1 Torr one whole day and night to give 130 g of a whey protein-soybean lecithin complex having a water content of 8.5% by weight

(Invention sample 5).

A whipped cream was prepared according to the same formulation and preparation process as those of Example 9 except that Invention sample 5 was used instead of Invention sample d, and the whipped cream containing Invnention sample 5 was examined for creamy flavor in the same manner as that of Example 9.

The results are given in Table 12. As shown in Table 12, the product containing Invention sample 5 was such a cream that it exhibited a stronger milky flavor and a better body and was free from the disagreeable tastes such as bitterness and astringent taste, as compared with the product not containing Invention sample 5.

Table 12

| Evaluation item | whole | milkiness | body | sweetness | disagreeable taste |
|---|---|---|---|---|---|
| Point | 0.8 | 0.6 | 0.7 | 0.55 | -0.4 |

(Example 27)

10 g of soybean protein was mixed with 5 g of phosphatidic acid, and 100 ml of water was added to the resulting mixture. The obtained mixture was dispersed and homogenized with a homogenizer. The obtained emulsion was spray-dried to give 14 g of a soybean protein-phosphatidic acid complex (Invention sample 6).

By adding Invention sample 6 to a powdered soup, the soup became free from disagreeable tastes, increased in body and improved in savor, and an excellent product was obtained.

(Example 28)

10 g of β-lactoglobulin was mixed with 4 g of phosphatidic acid, and 100 ml of water was added to the resulting mixture. The obtained mixture was dispersed and homogenized with a homogenizer. The obtained emulsion was freeze-dried to give 13 g of a β-lactoglobulin-phosphatidic acid complex (Invention sample 7).

Invention sample 7 was added to an aqueous solution (5 mM) of promethazine hydrochloride used as the raw material of pharmaceuticals to evaluate whether Invention sample 7 exhibits a bitterness-decreasing effect on it or not. More precisely, the intensity of the bitterness was evaluated according to the equivalent concentration method (see Example 1). For comparison, those containing, instead of Invention sample 7, sucrose, sorbitol and β-lactoglobulin, respectively which were used for the purpose of decreasing the bitterness in liquid preparations, were also evaluated in a similar manner to that described above. The results are given in Table 13.

As shown in Table 13, a solution of promazine hydrochloride having a decreased bitterness could be obtained by adding Invention sample 7.

Table 13

| Test substance | | Concn. of test substance | Intensity of bitterness |
|---|---|---|---|
| (1) | - (control) | - | 9.2 |
| (2) | Invention sample 7 | 0.3% | 5.0 |
| (3) | Invention sample 7 | 3% | 2.8 |
| (4) | sucrose | 20% | 7.1 |
| (5) | sorbitol | 30% | 6.9 |
| (6) | β-lactoglobulin | 3% | 9.0 |

Further, liquid preparations containing Invention sample 7 and one of pharmaceuticals A to G which will be described below were prepared in the same manner as that described above except that pharmaceuticals A to G were each used instead of the promethazine hydrochloride. The obtained liquid preparations were examined for taste. They were freed from the disagreeable bitterness inherent in pharmaceuticals and were easy to take orally.

A   quinone
B   chlorpromazine
C   papaverine
D   propranolol
E   berberine
F   brucine
G   strychnine

(Example 29)

10 of egg white albumin containing ovalbumin was kneaded with 4 g of phosphatidic acid and 2 g of phosphatidylinositol, and 100 ml of water was added to the obtained kneaded mixture. The obtained mixture was dispersed and homogenized by treating with a homogenizer. The obtained emulsion was dehydrated under the conditions of 20°C and 0.1 Torr one whole day and night to give 15 g of an albumin-phosphatidic acid-phosphatidylinositol complex (Invention sample 8).

1 g of acetaminophen was mixed with 20 g of Invention sample 8, and 100 ml of purified water was added th the obtained mixture, followed by agitation. Further, 5 g of synthetic aluminum silicate and mannitol were added thereto, and the obtained mixture was blended and vacuum-dried to give a powder. The bitterness of the powder thus obtained was compared with that of crude acetaminophen powder. This powder little tasted bitter.

(Example 30)

10 g of Invention sample 7 obtained in Example 28 was mixed with 0.5 g of promazine in a mortar to give a powder. This powder tasted less bitter than crude promazine powder.

(Example 31)

Invention sample 7 obtained in Example 28 was added to deionized water in a final concentration of 3.0%. Then, the obtained mixture was stirred to disperse Invention sample 7. The dispersion of β-lactoglobulin-phosphatidic acid complex thus obtained (Invention sample 9) was held in the mouth for about 10 seconds and then rinsed the oral cavity to fully spread the dispersion in the oral cavity. Thereafter, Invention sample 9 was spit out and a pharmaceutical having a bitter taste was held in the mouth to evaluate the bitter taste. For comparison, the intensity of the bitterness of the pharmaceutical was evaluated in the same manner as described above was repeated except that water or a 10% aqueous solution of sucrose was used instead of Invention sample 9. The evaluation of the intensity of the bitterness was conducted by ten healthy persons of both sexes in their twenties to forties according to the criteria which will be described below.

[Subjects of evaluation]

(1) Intensity of the bitterness of caffeine sensed when a 50 mM aqueous solution of caffeine is held in the mouth after 10-second hold of Invention sample 9 in the mouth and rinse of the mouth therewith.
(2) Intensity of the bitterness of caffeine sensed when a 50 mM aqueous solution of caffeine is held in the mouth after 10-second hold of water in the mouth and rinse of the mouth therewith.
(3) Intensity of the bitterness of caffeine sensed when a 50 mM aqueous solution of caffeine is held in the mouth after 10-second hold of a 10% aqueous solution of sucrose in the mouth and rinse of the mouth therewith.
(4) Intensity of the bitterness of quinine sensed when a 0.5 mM aqueous solution of quinine is held in the mouth after 10-second hold of Invention sample 9 in the mouth and rinse of the mouth therewith.
(5) Intensity of the bitterness of quinine sensed when a 0.5 mM aqueous solution of quinine is held in the mouth after 10-second hold of water in the mouth and rinse of the mouth therewith.
(6) Intensity of the bitterness of quinine sensed when a 0.5 mM aqueous solution of quinine is held in the mouth after 10-second hold of a 10% aqueous solution of sucrose in the mouth and rinse of the mouth therewith.

[Criteria for evaluation]

| 0 to 2 | not bitter |
|---|---|
| 3 to 4 | slightly bitter |
| 5 to 6 | somewhat bitter |
| 7 to 8 | strongly bitter |
| 9 to 10 | very strongly bitter |

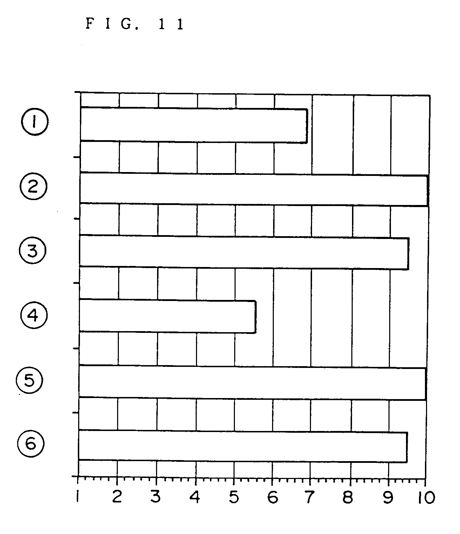

The results are given in Fig. 11. In Fig. 11, the averave values of the intensities of the bitterness are shown.
As is apparent from Fig. 11, Invention sample 9 which comprises an aqueous dispersion of Invention sample 7 exhibited a stronger bitterness-decreasing effect than that of an aqueous solution of sucrose which has hitherto been used as a bitterness-decreasing agent for pharmaceuticals.

(Example 32)

100 g of ovalbumin and 40 g of phosphatidic acid were dispersed in 1000 ml of water and then the obtained dispersion was homogenized with a homogenizer. The obtained emulsion was dehydrated under the conditions of 20°C and 0.1 Torr one whole day and night to give 130 g of an ovalbumin-phosphatidic acid complex (Invention simple 10). 50 g of Invention sample 10, 40 g of mannitol and 40 g of synthetic aluminum silicate were weighed and added to 500 ml of water. The obtained mixture was stirred and spray-dried to give 90 g of Invention sample 11 in the form of powder.
3 g of Invention sample 11 was held in the mouth and bitten for about 15 seconds. Thereafter, a 5 mM aqueous solution of promazine was held in the mouth. A less bitter taste was recognized as compared with the case wherein a 5 mM aqueous solution of promazine was held in the mouth without holding Invention sample 11 in the mouth.

## Claims

1. A taste-modifying agent characterised by comprising a protein-lipid complex which is an aggregate of a protein with a lipid.

2. The taste-modifying agent as claimed in claim 1, wherein the protein is water-soluble.

3. The taste-modifying agent as claimed in claim 1 or 2, wherein the lipid is a phospholipid, glycolipid, a sterol lipid, a fatty acid ester of polyol or a fatty acid.

4. The taste-modifying agent as claimed in any preceding claim wherein the protein-lipid complex is one prepared by homogeneously emulsifying and dispersing a protein and a lipid in water or other liquid and then effecting dehydration.

5. The taste-modifying agent according to any of claims 1 to 4 wherein the emulsification and homogenisation are carried out by mechanical means and/or sonication.

6. A bitterness-masking agent for pharmaceuticals, comprising a protein-lipid complex according to any preceding claim wherein the protein is of animal or vegetable origin.

7. A cosmetic characterised by including a taste-modifying agent according to any of claims 1 to 4.

8. A bitterness-decreasing agent characterised by comprising a protein-lipid complex which is an aggregate of an animal protein and/or a plant protein with a lipid.

9. The bitterness-decreasing agent as claimed in claim 8, wherein the lipid is a phospholipid.

10. The bitterness-decreasing agent as claimed in claim 8, wherein the animal protein and/or the plant protein are soluble in water.

11. The bitterness-decreasing agent as claimed in claim 9, wherein the protein-lipid complex is one prepared by homogeneously emulsifying and dispersing a protein and a lipid in water and then effecting dehydration.

12. A method for decreasing the perception of a bitter taste, characterised by introducing a taste modifying agent according to any of claims 1 to 4 into the oral cavity before and/or simultaneously with the oral ingestion of a bitter-tasting food, pharmaceutical or cosmetic so as to block the bitterness receptors present in the oral cavity.

13. A method for decreasing the bitterness of or modifying the taste of a foodstuff, pharmaceutical or cosmetic which comprises adding a taste-modifying agent according to any of claims 1 to 4 to the foodstuff, pharmaceutical or cosmetic.

14. A use of a taste-modifying agent according to any of claims 1 to 4 to decrease the bitterness of or modify the taste of foodstuff pharmaceutical or cosmetic.

15. A process for the preparation of a foodstuff, a medicinal composition or a cosmetic having a decreased bitterness which comprises adding a taste modifying agent according to any of claims 1 to 4 to a bitter-tasting food, pharmaceutical or cosmetic.

16. A food composition comprising a foodstuff and a taste modifying agent according to any of claims 1 to 4.

17. A cosmetic composition comprising a cosmetic component and a taste modifying agent according to any of claims 1 to 4.

18. A medical composition comprising a pharmaceutical and a taste modifying agent according to any of claims 1 to 4.

19. A medicinal composition as claimed in claim 18, wherein the pharmaceutical is a bitter-tasting one.

20. A medicinal composition as claimed in claim 18, wherein the protein-lipid complex is one prepared by using 0.01 to 100 parts by weight of lipid per part by weight of protein,

**Patentansprüche**

1. Geschmacksmodifizierendes Mittel,
   dadurch gekennzeichnet, daß
   es einen Protein-Lipid-Komplex umfaßt, der eine Zusammenlagerung aus einem Protein mit einem Lipid ist.

2. Geschmacksmodifizierendes Mittel nach Anspruch 1, wobei das Protein wasserlöslich ist.

3. Geschmacksmodifizierendes Mittel nach Anspruch 1 oder 2, wobei das Lipid ein Phospholipid, ein Glykolipid, ein Sterollipid, ein Polyol-Fettsäureester oder eine Fettsäure ist.

4. Geschmacksmodifizierendes Mittel nach einem der vorhergehenden Ansprüche, wobei der Protein-Lipid-Komplex durch homogene Emulgierung und Dispergierung eines Proteins und eines Fetts in Wasser oder einer anderen Flüssigkeit und anschließende Durchführung einer Dehydration hergestellt wird.

5. Geschmacksmodifizierendes Mittel nach einem der Ansprüche 1 bis 4, wobei die Emulgierung und Homogenisierung mit Hilfe mechanischer Mittel oder durch Beschallung durchgeführt werden.

6. Bitterkeit überdeckendes Mittel für Arzneimittel, umfassend einen Protein-Lipid-Komplex nach einem der vorhergehenden Ansprüche, wobei das Protein tierischen oder pflanzlichen Ursprungs ist.

7. Kosmetisches Mittel,
   dadurch gekennzeichnet, daß
   es ein geschmacksmodifizierendes Mittel nach einem der Ansprüche 1 bis 4 enthält.

8. Bitterkeit verminderndes Mittel,
   dadurch gekennzeichnet, daß
   es einen Protein-Lipid-Komplex umfaßt, der eine Zusammenlagerung eines tierischen Proteins und/oder eines pflanzlichen Proteins mit einem Lipid ist.

9. Bitterkeit verminderndes Mittel nach Anspruch 8, wobei das Lipid ein Phospholipid ist.

10. Bitterkeit verminderndes Mittel nach Anspruch 8, wobei das tierische Protein und/oder das pflanzliche Protein in Wasser löslich sind.

11. Bitterkeit verminderndes Mittel nach Anspruch 9, wobei der Protein-Lipid-Komplex durch homogene Emulgierung und Dispergierung eines Proteins und eines Lipids in Wasser und anschließende Durchführung einer Dehydration hergestellt wird.

12. Verfahren zur Verminderung der Empfindung eines bitteren Geschmacks,
    dadurch gekennzeichnet, daß
    ein geschmacksmodifizierendes Mittel nach einem der Ansprüche 1 bis 4 vor und/oder gleichzeitig mit der oralen Aufnahme eines bitter schmeckenden Lebensmittels, Arzneimittels oder kosmetischen Mittels in die Mundhöhle eingeführt wird, um die in der Mundhöhle vorhandenen Bitterkeits-Rezeptoren zu blockieren.

13. Verfahren zur Verminderung der Bitterkeit oder zur Modifizierung des Geschmacks eines Lebensmittels, Arzneimittels oder kosmetischen Mittels, umfassend die Zugabe eines geschmacksmodifizierenden Mittels nach einem der Ansprüche 1 bis 4 zu dem Lebensmittel, Arzneimittel oder kosmetischen Mittel.

14. Verwendung eines geschmacksmodifizierenden Mittels nach einem der Ansprüche 1 bis 4 zur Verminderung der Bitterkeit oder zur Modifizierung des Geschmacks eines Lebensmittels, Arzneimittels oder kosmetischen Mittels.

15. Verfahren zur Herstellung eines Lebensmittels, einer Arzneimittelzusammensetzung oder eines kosmetischen Mittels mit einer verminderten Bitterkeit, umfassend die Zugabe eines geschmacksmodifizierenden Mittels nach einem der Ansprüche 1 bis 4 zu einem bitter schmeckenden Lebensmittel, Arzneimittel oder kosmetischen Mittel.

16. Lebensmittelzusammensetzung, umfassend ein Lebensmittel und ein geschmacksmodifizierendes Mittel nach einem der Ansprüche 1 bis 4.

17. Kosmetische Zusammensetzung, umfassend einen kosmetischen Bestandteil und ein geschmacksmodifizierendes Mittel nach einem der Ansprüche 1 bis 4.

18. Arzneimittelzusammensetzung, umfassend ein Arzneimittel und ein geschmacksmodifizierendes Mittel nach einem der Ansprüche 1 bis 4.

19. Arzneimittelzusammensetzung nach Anspruch 18, wobei das Arzneimittel bitter schmeckt.

20. Arzneimittelzusammensetzung nach Anspruch 18, wobei der Protein-Lipid-Komplex unter Verwendung von 0,01 bis 100 Gewichtsanteilen Lipid pro Gewichtsanteil Protein hergestellt wird.

## Revendications

1. Agent de modification de goût, caractérisé en ce qu'il contient un complexe à base de protéines-lipides consistant en un agrégat d'une protéine avec un lipide.

2. Agent de modification de goût selon la revendication 1, caractérisé en ce que la protéine est soluble dans l'eau.

3. Agent de modification de goût selon la revendication 1 ou 2, caractérisé en ce que le lipide est un phospholipide, un glycolipide, un lipide stérolique, un ester d'acide gras de polyol ou un acide gras.

4. Agent de modification de goût selon l'une quelconque des revendications précédentes, caractérisé en ce que le complexe à base de protéines-lipides est préparé d'abord par mise en émulsion homogène et dispersion d'une protéine et d'un lipide dans l'eau ou dans un autre liquide puis par réalisation d'une déshydratation.

5. Agent de modification de goût selon l'une quelconque des revendications 1 à 4, caractérisé en ce que la mise en émulsion et l'homogénéisation sont réalisées par des moyens mécaniques et/ou par sonication.

6. Agent de masquage de l'amertume pour produits pharmaceutiques, contenant un complexe à base de protéines-lipides selon l'une quelconque des revendications précédentes, caractérisé en ce que la protéine est d'origine animale ou végétale.

7. Produit cosmétique caractérisé en ce qu'il contient un agent de modification du goût selon l'une quelconque des revendications 1 à 4.

8. Agent d'atténuation de l'amertume caractérisé en ce qu'il contient un complexe à base de protéines-lipides consistant en un agrégat d'une protéine animale et/ou d'une protéine végétale avec un lipide.

9. Agent d'atténuation de l'amertume selon la revendication 8, caractérisé en ce que le lipide est un phospholipide.

10. Agent d'atténuation de l'amertume selon la revendication 8, caractérisé en ce que la protéine animale et/ou la protéine végétale sont solubles dans l'eau.

**11.** Agent d'atténuation de l'amertume selon la revendication 9, caractérisé en ce que le complexe à base de protéines-lipides est préparé d'abord par mise en émulsion homogène et dispersion d'une protéine et d'un lipide dans l'eau puis par réalisation d'une déshydratation.

**12.** Procédé d'atténuation de la perception d'un goût amer, caractérisé par l'introduction d'un agent de modification du goût selon l'une quelconque des revendications 1 à 4 dans la cavité buccale avant et/ou simultanément à l'ingestion orale d'un aliment, d'un produit pharmaceutique ou d'un produit à visée cosmétique présentant un goût amer, de façon à bloquer les récepteurs d'amertume présents dans la cavité buccale.

**13.** Procédé d'atténuation de l'amertume ou de modification du goût d'un produit alimentaire, pharmaceutique ou cosmétique comprenant l'ajout d'un agent de modification du goût selon l'une quelconque des revendications 1 à 4 au produit alimentaire, pharmaceutique ou cosmétique.

**14.** Utilisation d'un agent de modification du goût selon l'une quelconque des revendications 1 à 4 afin d'atténuer l'amertume ou de modifier le goût d'un produit alimentaire, pharmaceutique ou cosmétique.

**15.** Procédé de fabrication d'un produit alimentaire, pharmaceutique ou cosmétique présentant une amertume atténuée comprenant l'ajout d'un agent de modification du goût selon l'une quelconque des revendications 1 à 4 à un produit alimentaire, pharmaceutique ou cosmétique présentant un goût amer.

**16.** Composition alimentaire comprenant un produit alimentaire et un agent de modification du goût selon l'une quelconque des revendications 1 à 4.

**17.** Composition cosmétique comprenant un composant cosmétique et un agent de modification du goût selon l'une quelconque des revendications 1 à 4.

**18.** Composition médicinale comprenant un produit pharmaceutique et un agent de modification du goût selon l'une quelconque des revendications 1 à 4.

**19.** Composition médicinale selon la revendication 18, caractérisée an ce que le produit pharmaceutique est un produit présentant un goût amer.

**20.** Composition médicinale selon la revendication 18, caractérisée en ce que le complexe à base de protéines-lipides est préparé au moyen de 0,01 à 100 parties en poids de lipide par partie en poids de protéine.

F I G.  1

Determination of sweetness (sucrose) by
equivalent concentration method

F I G.  2

Determination of umami (sodium glutamate)
by equivalent concentration method

F I G. 3

Determination of bitterness (quinine
sulfate) by equivalent concentration method

F I G. 4

Determination of sourness (citric anhydride)
by equivalent concentration method

F I G. 5

Determination of saltiness (sodium chloride)
by equivalent concentration method

F I G. 6

Change in membrane potential $\Delta$ F (%)

FIG. 7

FIG. 8

F I G. 9

FIG. 10

FIG. 11